(19) **Europäisches Patentamt European Patent Office Office européen des brevets**

(11) **EP 4 534 646 A1**

(12) **EUROPEAN PATENT APPLICATION**
published in accordance with Art. 153(4) EPC

(43) Date of publication:
09.04.2025 Bulletin 2025/15

(21) Application number: 23811230.4

(22) Date of filing: 26.05.2023

(51) International Patent Classification (IPC):
C12N 1/18 (2006.01)      C12N 1/38 (2006.01)
C12N 15/01 (2006.01)      C12G 1/02 (2006.01)
C12G 3/02 (2019.01)      C12R 1/865 (2006.01)

(52) Cooperative Patent Classification (CPC):
C12G 1/02; C12G 3/02; C12N 1/18; C12N 1/38;
C12N 15/01; C12R 2001/865

(86) International application number:
PCT/ES2023/070344

(87) International publication number:
WO 2023/227820 (30.11.2023 Gazette 2023/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.05.2022 ES 202230456

(71) Applicants:
• **Universitat de Valéncia**
  **46010 Valencia (ES)**
• **Consejo Superior De Investigaciones Científicas (CSIC)**
  **28996 Madrid (ES)**

(72) Inventors:
• **ARANDA FERNÁNDEZ, Agustín**
  **46980 Paterna (València) (ES)**
• **MATALLANA REDONDO, Emilia**
  **46980 Paterna (València) (ES)**
• **GARRIGÓS CONTELLES, Víctor**
  **46980 Paterna (València) (ES)**

(74) Representative: **Cueto, Sénida**
  **SP3 Patents S.L.**
  **Los Madroños, 23**
  **28891 Velilla de San Antonio (ES)**

Remarks:
The complete document including Reference Table(s) and the Sequence Listing(s) can be downloaded from the EPO website

(54) **YEAST STRAINS WITH OPTIMISED WINE-MAKING PROPERTIES AND PRODUCTION METHOD THEREOF**

(57)    The present invention relates to a method for producing improved strains of *Saccharomyces cerevisiae* with optimised wine-making properties, which comprises preparing at least one culture, wherein an initial strain of *S. cerevisiae* is in contact with at least 10 mg/l of 2-aminoethyl-L-cysteine in a culture medium and is grown for at least five weeks; and isolating individual mutants of the *S. cerevisiae* strain with optimised wine-making properties once that time has elapsed. The invention also relates to improved strains obtained using the method and to uses of same in the alcoholic fermentation of vegetable substrates.

EP 4 534 646 A1

FIG. 7

FIG. 7 (Continuation)

**Description**

**Field of the invention**

The present invention relates to a method for obtaining non-genetically modified yeast strains with optimized wine-making properties, as well as to said strains.

**State of the art**

The traditional way of winemaking relies on the spontaneous fermentation of grape must by microorganisms present on the surface of the grapes and in the equipment and environment of the winery. These wines are organoleptically complex and better reflect the environment (*terroir*), but the process is less predictable and reproducible. For this reason, modern oenology uses yeast starter cultures of proven efficiency. Although there are yeasts from other species that provide nuances to the wine, the bulk of winemaking is carried out by yeasts of the genus *Sacccharomyces,* particularly the species S. *cerevisiae.* This is due to its great fermentation efficiency, so that it produces a large amount of ethanol even if oxygen is present, due to the so-called Crabtree effect, and its great tolerance to stress (Matallana E, Aranda A. Biotechnological impact of stress response on wine yeast. Lett Appl Microbiol. 2017;64(2):103-110. doi:10.1111/lam.12677).

The bulk of the metabolism of the sugars present in the must is dedicated to the production of ethanol through fermentation, so that the pyruvate produced by glycolysis is not respired via mitochondria, but is decarboxylated to $CO_2$ and acetaldehyde, which is used to reoxidize the reduced equivalents, producing ethanol, and with a low production of yeast biomass. Only when sugar levels are very low is respiration activated, producing much more biomass and energy in the form of ATP per mole of glucose consumed. Part of the NADH in fermentation is diverted for the production of glycerol, a polyalcohol essential for tolerance to hyperosmotic stress, and which gives the wine characteristic body and sweetness, constituting the third component by weight of the wine, after water and ethanol. Part of the acetaldehyde can be diverted in the production of acetic acid, which on the contrary is an undesirable product since it contributes to volatile acidity, and gives a vinegary character to the wine as soon as a threshold is crossed. Thus, carbohydrate metabolism and the balance between fermentation and respiration are essential for the production of wine with a correct organoleptic balance. Global warming results in an increase in the sugar content of the grape must, with a consequent increase in ethanol levels. Therefore, any way to stop the increase in alcohol content is important in oenology, and diverting the metabolic flow from ethanol to glycerol is a very attractive possibility. Reducing the alcohol level results in healthier products that are appreciated by the younger and female consumers.

Over the years, a multitude of strains of *S. cerevisiae* from diverse geographical origins have been selected that combine good fermentative performance with a correct organoleptic profile, but there is a certain degree of variation with respect to fermentative parameters such as adaptation to extreme temperatures, speed of fermentation, nitrogen requirements, etc. Therefore, the winemaker has a palette of strains to choose from in each condition, but there is no ideal strain for all musts and types of wine, since those that have good tolerance to stress or low nutritional requirements may not be the ones. that provide more aromas, so there are many yeast strains that can potentially be improved in one or more aspects. Starter cultures are generally sold in dehydrated form, as Active Dry Yeast (ADL), which results in a stable product. Biomass propagation is carried out with beet or sugar cane molasses under fed-batch conditions with aeration to avoid the Crabtree effect. The biomass thus generated is dried in a fluidized bed dehydrator.

The stress response is conditioned by the physiological state of the cell and the food availability (Orozco, Matallana and Aranda Stress Response in Yeasts Used for Food Production. Food Molecular Microbiology. Taylor & Francis 2019). The connection between stress response and metabolism is controlled by Nutrient Signaling Pathways, so that with abundant nutrients, yeast promotes growth and proliferation and mitigates the stress response. The stresses and their response during winemaking are of metabolic nature, so that the high amount of glucose and fructose in the must produces a hyperosmotic stress that is compensated with the production of the compatible osmolyte glycerol. At the end of fermentation, the two-carbon metabolites, ethanol, acetic acid and acetaldehyde are toxic molecules that promote cellular aging. Manipulation of these pathways can be used to modulate fermentative behavior, altering the production of key metabolites.

One relevant pathway is the so-called Retrograde Response (RR). This pathway responds to the metabolic state of the mitochondria and communicates it to the cell nucleus, regulating the expression of enzymes that act in the mitochondria (such as several involved in the tricarboxylic acid cycle) and the peroxisome in order to provide the carbon skeletons necessary for the biosynthesis of certain amino acids, such as lysine and glutamic acid. Therefore, activation of the pathway grants tolerance to a toxic analogue of lysine, 2-aminoethyl-L-cysteine, Ramos,. F., Wiame. JM. Mutation affecting the specific regulatory control of lysine biosynthetic enzymes in Saccharomamyces cerevisiae. Molec Gen. Genet. 200,. 291-294 (1985). https://doi.org/10.0007/BF00425438 This pathway also stimulates the anaplerotic reaction of pyruvate carboxylase, so that part of the pyruvate from glycolysis is diverted to be used as the carbon skeleton of various amino acids. Such a role in the synthesis of nitrogen compounds makes the retrograde response related to the main amino acid response pathway, the TORC1 complex.

In the doctoral thesis Analysis of nutrient signaling pathways in wine strains of Saccharomyces cerevisiae under winemaking conditions by Beatriz Vallejo Estará (2019), it is described that the activation of the Retrograde Response in wine yeasts by genetic manipulation of its repressor MKS1 causes an increase in the production of glycerol and a decrease in ethanol and acetic acid. However, these strains have the disadvantage that with the current regulation they cannot be marketed as they are considered transgenic microorganisms. However, the strains obtained by the method of the invention present at least one alteration in the nucleotide sequence of the RTG2 gene that has as a result the decrease in acetic acid and the increase in glycerol in a wine fermentation process

The doctoral thesis Analysis of nutrient signaling pathways in wine strains of Saccharomyces cerevisiae under winemaking conditions by Beatriz Vallejo Estará (2019) discloses a directed evolution procedure of the S. *cerevisiae* strain M2 using 2-deoxyglucose and rapamycin respectively. This document does not disclose the use of 2-AEC for the same purpose. Furthermore, strain M2 evolved in the presence of rapamycin or 2-deoxyglucose does not present an increase in glycerol production capacity.

The document Fit-for-purpose yeast and bacteria via directed evolution (https:/7www.wineaustralia.com/research/projects/fit-for-purpose-yeast-and-bacteria-via-d) discloses the method of improving strains of yeast for wine production. However, it does not disclose the use of the toxic lysine analogue 2-aminoethyl-L-cysteine (2-AEC), in a directed evolution procedure. Specifically, conditions of low pH and high ethanol concentration are used to direct the procedure.

Patent US10829827B2 describes a method of obtaining yeast strains with lower ethanol production through a directed evolution process in culture medium with increasing concentrations of salt causing hyperosmotic stress. This document does not disclose the use of 2-AEC and, furthermore, the method of the present invention is faster since the improved strains are obtained with a smaller number of generations.

The scientific article by Gasent-Ramírez JM, Benitez T. Lysine-overproducing mutants of Saccharomyces cerevisiae baker's yeast isolated in continuous culture. Appl Environ Microbiol. 1997;63(12):4800-4806. doi:10.1128/aem.63.12.4800-4806.1997; discloses a procedure for obtaining mutants of S. *cerevisiae* with high lysine production by exposure to increasing concentrations of the toxic lysine analogue 2-AEC. Although this document discloses the use of the 2-AEC compound for a method of directed evolution, it does not indicate that it can be used to improve the wine properties of S. *cerevisiae* strains: It only indicates baking and does not mention at any time whether these mutants can produce more glycerol or less acetic acid.

## Description

The present specification discloses a method to obtain mutant strains by adaptive evolution, not genetically modified, that are able, when compared with the strain of origin, of altering the production of compounds of oenological interest derived from fermentative metabolism, such as glycerol, acetic acid and/or ethanol during wine fermentation. The process is based on the use of the chemical compound 2-aminoethyl-L-cysteine, since the mutants that tolerate this compound have the Retrograde Response activated, giving rise to the desired phenotype.

In the present report 'mutant strains' and 'improved strains' are synonymous and are used interchangeably.

In the present report 'AEC', '2AEC', '2-AEC', '2-aminoethyl-L-cysteine' and 'S-(2-Aminoethyl)-L-cysteine hydrochloride' are synonyms and are used interchangeably and refer to a toxic analogue of the amino acid lysine.

In this report "vinification" and "alcoholic fermentation of grape must" are synonyms and are used interchangeably.

In the present report "directed evolution" and "adaptive evolution" are synonyms and are used interchangeably.

The object of the present invention refers to a method of obtaining an improved strain of *Saccharomyces cerevisiae* with optimized wine-making properties that comprises the following steps:

a) Growing an initial strain of S. *cerevisiae* in a culture medium with a concentration of 2-aminoethyl-L-cysteine from 10 mg/l to 90 mg/l of culture medium for at least 5 weeks.

b) isolating single mutants of the improved strain of S. *cerevisiae* with optimized wine-making properties after the time of step a)

where optimized wine-making properties refer to higher glycerol production and lower acetic acid production compared to the initial strain of the which it comes when a grape must vinification process is performed.

In a preferred particular embodiment, the period of time required is at least 6 weeks, more preferably at least 7 weeks, still more preferably at least 8 weeks, still more preferably at least 9 weeks, still more preferably at least 10 weeks.

In a particular embodiment the period of time required is between 6 and 40 weeks, preferably between 7 and 35 weeks, more preferably between 8 and 30 weeks, still more preferably between 9 and 25 weeks and still more preferably between 10 and 20 weeks.

In another particular embodiment, the amount of 2-aminoethyl-L-cysteine is between 10 mg/l and 90 mg/l of culture medium, preferably between 13 mg/l and 85 mg/l, more preferably between 15 mg/l and 80 mg/l, still more preferably between 20 mg/l and 65 mg/l, still more preferably between 30 mg/l and 40 mg/l.

The 2-aminoethyl-L-cysteine concentrations indicated in the previous paragraph are equivalent to between 50 $\mu$M and 449 $\mu$M, preferably between 65 $\mu$M and 424 $\mu$M, more preferably between 75 $\mu$M and 399 $\mu$M, still more preferably between 100 $\mu$M and 324 $\mu$M, still more preferably between 150 $\mu$M and 200 $\mu$M.

In another particular embodiment the required period of time is between 8 and 12 weeks and the amount of 2-aminoethyl-L-cysteine is between 30 mg/l and 40 mg/l of culture medium.

Prior to step a) a colony of the initial strain can be grown in a pre-culture without 2-AEC.

The method of the invention can be carried out in both continuous and discontinuous culture (*batch*).

The advantage of a discontinuous or batch culture is that population bottlenecks are created that can favour certain specific mutations.

In a particular embodiment, when the culture is discontinuous and when the OD(600nm) of the previous culture has reached the stationary phase, this is, an optical density OD(600nm) higher than 4, a fraction of said culture is inoculated into a new culture with culture medium and fresh 2-aminoethyl-L-cysteine.

When the method of the invention is performed on a batch culture, it is necessary to measure the optical density (OD(600nm)). When it exceeds the value of 4, preferably 4.5, more preferably 5, implying that the culture is in stationary phase, a fraction of said culture is (re)inoculated with a new culture so that the initial density OD(600nm) of the new culture is between 0.05 and 0.2, preferably, 0.08 and 0.15, to start a new cycle. This happens approximately every 2 or 3 days. In a period of 5 weeks, between 14 and 15 re-inoculations or passes will have to be carried out. The same or different concentration of 2AEC that was being used will also have to be added to the new culture, for example, between 10 mg/l and 90 mg/l.

In a particular embodiment, the concentration of 2-aminoethyl-L-cysteine is kept constant throughout the entire time period, this means that the amount of 2AEC does not increase or decrease in each new culture.

The determination of the number of generations was made from the data obtained from the optical density (600nm) of each of the passes performed, when the method of the invention is carried out in a batch culture.

$$\text{RATIO} = (\text{final OD600}) / (\text{initial OD600})$$

$$\text{Generations} = \log_2(\text{RATIO})$$

$$\text{Accumulated generations} = \text{sum of generations in each pass}$$

The method for calculating the generations was obtained from the following document: Xu, X., Williams, T. C., Divne, C., Pretorius, I. S., Paulsen, I. T. (2019). Evolutionary engineering in Saccharomyces cerevisiae reveals a TRK1-dependent potassium influx mechanism for propionic acid tolerance. Biotechnol Biofuels 12, 97 (2019). https://doi.org/10.1186/s13068-019-1427-6

A period of 5 weeks is the period of time necessary to complete approximately 75 generations of said initial strain.

In a particular embodiment, the required period of time is at least 80 generations, more preferably at least 90 generations, still more preferably at least 100 generations, still more preferably at least 120 generations, still more preferably at least 130 generations, still more preferably at least 140 generations, still more preferably at least 150 generations.

In a particular embodiment the maximum required period of time is at least 300 generations, more preferably at least 250 generations, still more preferably at least 220 generations, still more preferably at least 200 generations, still more preferably at least 180 generations, still more preferably at least 170 generations, still more preferably at least 160 generations.

In another particular embodiment the required period of time is between at least 120 generations and 180 generations, i.e. between 8 weeks and 12 weeks.

The environmental conditions of the method of the invention are the same as those used for growth cultures of S. *cerevisiae* strains which are common in the state of the art and a person skilled in the art would know how to find the ideal conditions.

The growth temperature can be between 10°C and 60°C, preferably between 15°C and 50°C, more preferably between 20°C and 40°C and even more preferably between 25°C and 35°C.

The agitation of the batch culture, may be between 10 and 400 revolutions per minute (rpm), preferably between 50 and 300 rpm, more preferably between 100 and 250 rpm, more preferably between 150 and 220 rpm, and still more preferably between 160 and 200 rpm.

In a continuous culture the agitation is different as it occurs by means of propellers located inside the bioreactor, this agitation can be between 100 and 700 rpm, preferably between 200 and 600 rpm, more preferably between 300 and 500 rpm.

By way of example, the culture of the *S. cerevisiae* strain in contact with 2-AEC can be grown in batch, at a temperature between 15°C and 45°C, preferably 20°C and 40°C, more preferably between 25°C and 35°C.

In another particular embodiment, where the culture medium is selected between SD (Synthetic defined), SC (Synthetic Complete) minimal medium without lysine and MS300 synthetic must without lysine, or any minimal medium without amino acids. A skilled person would know how to prepare any of these media since it is a routine task in the field. As an example, the culture media from: Dymond JS. Saccharomyces cerevisiae growth media. Methods Enzymol. 2013;533:191-204. doi: 10.1016/B978-0-12-420067-8.00012-X

In a preferred embodiment of the method of the invention the S. *cerevisiae* strain is contacted with 2-AEC in a batch culture with SD medium at a temperature between 25°C and 35°C with agitation between 160 rpm and 200 rpm. When the OD(600nm) exceeds a value of 5, a fraction of this culture is (re)inoculated into a new culture so that the density in fresh SD medium at an initial OD(600nm) is 0.1, continuing for at least 150 generations to 180 generations, i.e. 8 to 12 weeks and the amount of 2-aminoethyl-L-cysteine is between 30 mg/L and 40 mg/L.

With regard to step b) of the method of the invention, at the end of the selection process individual colonies can be isolated on non-selective rich medium, e.g. YPD/YEPD (Yeast Extract, Peptone, Dextrose) and their tolerance to 2-AEC was tested by serial dilution droplets on plates of SD selective medium with 2-AEC at a concentration of between 30 mg/L and 40 mg/L.

In a particular embodiment of step b) of the method of the invention, isolation of mutants of the improved S. *cerevisiae* strain can be performed by serial dilutions and seeding them directly on SD selective medium plates with 2-AEC at a concentration between 30 mg/L and 40 mg/L.

Serial dilutions can be performed in water, saline or SD medium.

One of the advantages of the method of the invention is reproducibility as improved S. *cerevisiae* strains with optimised winemaking properties have been obtained from 3 commercial yeast strains with a different genetic background.

The mutants with higher tolerance to 2-AEC were used for small-scale fermentations in grape must to determine the improvement of the production of chemical compounds of oenological interest, i.e. with optimised winemaking properties. Optimised winemaking properties refers to 2 or more of the following:

- higher glycerol production,
- lower acetic acid production, and
- lower ethanol production

compared to the initial strain from which it comes, when a grape must vinification process is carried out.

In a particular embodiment the grape must can be natural, synthetic and combinations of both.

The differences with respect to the document by Gasent-Ramírez JM is that it allows the generation of strains with improved winemaking properties that would not optimal for bakery, since the characteristics mentioned in the previous paragraph give rise to a lower production of $CO_2$ by diverting the glycolytic flow to glycerol production. Gaseous $CO_2$ is essential in baking and confectionery so that the products acquire their spongy texture, so the strains of the present invention would not be suitable in confectionery since they would require more time to give rise to the confectionery products than the parent strains. This document describes the obtaining of strains that produce more lysine and does not describe the increase in glycerol or the decrease in acetic acid or ethanol.

Controlling the amount of acetic acid is important for the organoleptic characteristics of the wine, since very high levels of this compound make it vinegary, thus reducing its quality.

Glycerol, on the other hand, has a positive effect on the quality of wine. It is not aromatic, due to its non-volatile nature, but contributes to the density, body, softness, and texture of wines.

In a particular embodiment, optimized wine-making properties refers to improved strains with higher glycerol production and lower acetic acid production compared to the initial strain.

In another particular embodiment, optimized wine-making properties refers to improved strains with higher glycerol production, lower acetic acid production and lower ethanol production compared to the initial strain.

In a particular embodiment, the glycerol production of the strain with optimized wine-making properties compared to the initial strain from which it comes is at least 10% (w/v) more, preferably at least 20% (w/v). ) more, more preferably at least 30% (w/v) more, even more preferably at least 40% more, even more preferably at least 50% (w/v) more, even more preferably at least 60% (w/v) more, even more preferably at least 70% (w/v) more, even more preferably at least 80% (w/v) more, even more preferably at least 90% (w/v) more and even more preferably at least 100 (w/v) more when a grape must vinification process is carried out.

That is, the product obtained from a grape must vinification process with a strain with optimized wine-making properties has at least 10% (w/v) more glycerol than the product obtained from a vinification process of grape must with the initial strain from which it comes, preferably at least 20% (w/v) more, more preferably at least 30% (w/v) more, even more preferably at least 40% (w/v) more, even more preferably at least 50% (w/v) more, even more preferably at least 60% (w/v) more, even more preferably at least 70% (w/v) more, even more preferably at least 80% (w/v) more, even more preferably at least 90% (w/v) more and even more preferably at least 100% (w/v) more glycerol, compared to the glycerol content (w/v) of the product of a grape must vinification process carried out under the same conditions but with the initial strain from which the strain with optimized wine-making properties has been obtained.

In another particular embodiment, the ethanol production of the strain with optimized wine-making properties compared to the initial strain from which it comes is at least 0.5% (v/v) less, preferably at least 2% (v/v) less, more preferably at least 5% (v/v) less, even more preferably at least 8% (v/v) less, even more preferably at least 10% (v/v) less, even more preferably at least 15% (v/v) less, even more preferably at least one 20% (v/v) less, even more preferably at least 30% (v/v) less, and even more preferably at least 50% (v/v) less when a grape must vinification process is carried out.

That is, the product obtained from a grape must vinification process with a strain with optimized wine-making properties has at least 0.5% (v/v) less ethanol than the product obtained from a vinification process of the grape must with the initial strain from which it comes, preferably at least 2% (v/v) less, more preferably at least 5% (v/v) less, even more preferably at least 8% (v/v) less, even more preferably at least 10% (v/v) less, even more preferably at least 15% (v/v) less, even more preferably at least 20% (v/v) less, even more preferably at least 30% (v/v) less, and even more preferably at least 50% (v/v) less when a grape must vinification process is carried out, compared to the ethanol content (v/v) of the product of a grape must vinification process carried out under the same conditions but with the initial strain from which the strain with optimized wine-making properties has been obtained.

The reduction in ethanol production has been observed in the evolved strains eM2c and eT73I, which have homozygous mutations in RTG2, although it is expected that the mutation in *LYS21* in the eT73I strain is also involved, as they share a metabolic pathway.

In another particular embodiment, the acetic acid production of the strain with optimized wine-making properties compared to the initial strain from which it comes is at least 0.5% (w/v) less, preferably at least 2% ( w/v) less, more preferably at least 5% (w/v) less, even more preferably at least 10% (w/v) less, even more preferably at least 20% (w/v) less, even more preferably at least 40% (w/v) less, even more preferably at least 50% (w/v) less, even more preferably at least 60% (w/v) less, even more preferably at least one 70% (w/v) less, and even more preferably at least 80% (w/v) less when a grape must vinification process is carried out.

That is, the product obtained from a grape must vinification process with a strain with optimized wine-making properties has at least 0.5% (w/v) less acetic acid than the product obtained from a grape must vinification process with the initial strain from which it comes, preferably at least 2% (w/v) less, more preferably at least 5% (w/v) less, even more preferably at least 10% (w/v) less, even more preferably at least 20% (w/v) less, even more preferably at least 40% (w/v) less, even more preferably at least 50% (w/v) less, even more preferably at least 60% (w/v) less, even more preferably at least 70% (w/v) less, and even more preferably at least 80% (w/v) less when a grape must vinification process is carried out compared to the acetic acid content (w/v) of the product of a grape must vinification process carried out under the same conditions but with the initial strain from which the strain with optimized wine-making properties has been obtained.

In another particular embodiment, the strain with optimized wine-making properties has a reduction of at least 20% (w/v) in the production of acetic acid and an increase of at least 60% (w/v) in the production of glycerol in a vinification process compared to the same strain without carrying out the method of the invention.

In another particular embodiment, the strain with optimized wine-making properties has a reduction of at least 5% (v/v) in the production of ethanol, a reduction of at least 20% (w/v) in the production of acetic acid and an increase of at least 60% (w/v) in glycerol production in a winemaking process compared to the same strain without carrying out the method of the invention.

In a particular embodiment of the method of the invention, the *Saccharomyces cerevisiae* strain with optimized wine-making properties has one or more non-synonymous mutations in the nucleotide sequence of the *RTG2* gene.

Non-synonymous mutations are those that modulate the activity of the protein encoded by the *RTG2* gene.

In another embodiment of the invention the mutation is homozygous or heterozygous.

In the present specification, the *RTG2* Gene ID is 852640 in GenBank and the UniProt code of the protein is P32608.

*RTG2* is a transcriptional regulator of the nutrient signalling pathway known as the Retrograde Response. This pathway promotes the synthesis of amino acids such as lysine and glutamine from carbon skeletons derived from pyruvate produced during glycolysis.

The technical effect of mutations in the nucleotide sequence of the *RTG2* gene is that they reduce ethanol and acetic acid and increase glycerol when *Saccharomyces cerevisiae* strains undergo wine fermentation, i.e. they show optimised wine-making properties.

In another particular embodiment, the mutations in the nucleotide sequence of the *RTG2* gene result in one or more of the following amino acid mutations of the resulting protein R30C; G248E and R560I.

In another embodiment of the method of the invention the *Saccharomyces cerevisiae* strain with optimised wine-making properties has one or more non-synonymous mutations in the nucleotide sequence of the homocitrate synthase genes *LYS20* and *LYS21.*

In the present specification, the *LYS20* Gene ID is 851346 in GenBank and the UniProt code for the protein is P48570.

In the present specification, the *LYS21* Gene ID is 851425 in GenBank and the UniProt code of the protein is Q12122.

Lys20 and its paralogue Lys21 form the homocitrate synthase, which catalyses the condensation of $\alpha$-ketoglutarate and acetyl-CoA to give homocitrate, the first step in lysine synthesis. This mutation could lead to a hyperactivated or retroinhibition-insensitive enzyme that diverts metabolic flux from pyruvate to the production of the amino acid lysine by reducing pyruvate and thus glycolytic flux, favouring glycerol production and also consuming acetyl-CoA and thus decreasing acetic acid. *RTG2* controls *LYS20* and *LYS21* and more *LYS* genes, so the more activating mutations there are, the more active the pathway is, favouring glycerol production and decreasing acetic acid.

Non-synonymous mutations are those that modulate the activity of the proteins encoded by the *LYS20* and *LYS21* genes.

In another embodiment of the invention the mutation is homozygous or heterozygous.

In another particular embodiment, mutations in the nucleotide sequence of the *LYS20* and *LYS21* genes give rise to one or more of the following amino acid mutations of the resulting protein N379D and R390G respectively.

The technical effect of the mutations in *LYS21, RTG2* and *LYS20,* in addition to obtaining optimized wine-making properties, is the activation of lysine metabolism and increasing tolerance to AEC.

In another particular embodiment, the strains obtained by the method of the invention present an increase in the expression level of the *CIT2* and *DLD3* genes with respect to the initial strain under exponential growth conditions in complete, nutrient-rich medium, which indicates that retrograde response is activated

In the present specification, the *CIT2* Gene ID is 850361 in GenBank and the UniProt code of the protein is P08679.

In the present specification, the *DLD3* Gene ID is 856638 in GenBank and the UniProt code of the protein is P39976.

Another additional object of the invention refers to the use of one or more of the mutant alleles in *RTG2, LYS20* and *LYS21* as markers to detect a strain with optimised wine-making properties by the method of the invention described above.

Another additional object of the invention refers to the use of one or more of the mutant alleles in *RTG2, LYS20* and *LYS21* as tools for improving the wine-making properties of any yeast of the *S. cerevisae* species into which they are introduced.

In a particular embodiment the *RTG2* mutant alleles are selected from one or more of R30C; G248E and R560I

In another particular embodiment the mutant allele of *LYS20* is N379D.

In another particular embodiment the mutant allele of *LYS21* is R390G.

A further object of the present invention relates to a strain of *Saccharomyces cerevisiae* with optimized wine-making properties obtained by the method described above.

Optimized wine-making properties refers to 2 or more of the following: higher glycerol production, lower acetic acid production, and lower ethanol production compared to the initial strain from which it comes, when a subsequent grape must vinification process is carried out

In a particular embodiment, optimized wine-making properties refers to improved strains with higher glycerol production and lower acetic acid production compared to the initial strain.

In another particular embodiment, optimized wine-making properties refers to improved strains with higher glycerol production, lower acetic acid production and lower ethanol production compared to the initial strain.

In another particular embodiment, the *Saccharomyces cerevisiae* strain with optimized wine-making properties has one or more mutations in the nucleotide sequence of the *RTG2* gene.

In another particular embodiment, mutations in the nucleotide sequence of the *RTG2* gene give rise to one or more of the following amino acid mutations of the resulting R30C protein; G248E and R560I.

In another particular embodiment, the *Saccharomyces cerevisiae* strain with optimized wine-making properties has one or more mutations in the nucleotide sequence of the homocitrate synthase genes *LYS20* and *LYS21.*

In another particular embodiment, the mutation is homozygous or heterozygous.

In another particular embodiment, mutations in the nucleotide sequence of the *LYS20* and *LYS21* genes give rise to one or more of the following amino acid mutations of the resulting protein N379D and R390G respectively.

The present invention also refers to a strain of *Saccharomyces cerevisiae* deposited in the Spanish Type Culture Collection with number 13204 or mutants derived from it, where said mutants have the same or better wine-making properties than the CECT13204 strain.

This strain was deposited on May 13, 2022 in the Spanish Type Culture Collection, Calle Catedrático Agustin Escardino, 9, CP46980, Paterna (Valencia), Spain, under the provisions of the Treaty of Budapest.

In the present specification the strain "CECT13204" and "eM2c" are interchangeable terms and are used indistinctly.

Mutants derived from this strain refer to strains that are derived from CECT13204, or obtained from CECT13204, and may have mutations compared to *Saccharomyces cerevisiae,* preferably, the mutant strain has the same or better wine-making properties than the initial strain CECT13204. Preferably, the mutant derived from CECT13204 has at least 80% or more, preferably at least 90% or more, more preferably at least 95% or more or even up to 100% or more than 100% of the optimized wine-making properties compared to the CECT13204 strain under equal conditions.

The present invention also refers to a strain of *Saccharomyces cerevisiae* deposited in the Spanish Type Culture Collection with number 13205 or mutants derived from it, where said mutants have the same or better wine-making properties than the CECT13205 strain.

This strain was deposited on May 13, 2022 in the Spanish Type Culture Collection, Calle Catedrático Agustin Escardino, 9, CP46980, Paterna (Valencia), Spain under the provisions of the Treaty of Budapest.

In the present invention the strain "CECT13205" and "eT73I" are interchangeable terms and are used indistinctly.

Mutants derived from this strain refer to strains that are derived from CECT13205, or obtained from CECT13205, and may have mutations compared to *Saccharomyces cerevisiae,* preferably, the mutant strain has the same or better wine-making properties than the initial strain CECT13205. Preferably, the mutant derived from CECT13205 has at least 80% or more, preferably at least 90% or more, more preferably at least 95% or more or even up to 100% or more than 100% of the optimized wine-making properties compared to strain CECT13205 under equal conditions.

The present invention also refers to a strain of Saccharomyces cerevisiae deposited in the Spanish Type Culture Collection with number 13206 or mutants derived from it, where said mutants have the same or better wine-making properties than the CECT13206 strain.

This strain was deposited on May 13, 2022 in the Spanish Type Culture Collection, Calle Catedrático Agustin Escardino, 9, CP46980, Paterna (Valencia), Spain under the provisions of the Treaty of Budapest.

In the present invention the strain "CECT13206" and "eEC1118o" are interchangeable terms and are used indistinctly.

Mutants derived from this strain refer to strains that are derived from CECT13206, or obtained from CECT13206, and may have mutations compared to *Saccharomyces cerevisiae,* preferably, the mutant strain has the same or better wine-making properties than the initial strain CECT13206. Preferably, the mutant derived from CECT13206 has at least 80% or more, preferably at least 90% or more, more preferably at least 95% or more or even up to 100% or more than 100% of the optimized wine-making properties compared to the CECT13206 strain under equal conditions.

The optimized wine-making properties of strains CECT13204, CECT13205 and CECT13206 refer to 2 or more of the following:

- higher glycerol production,
- lower acetic acid production, and
- lower ethanol production

compared to the initial strain from which it comes, when A grape must vinification process is carried out.

In a particular embodiment, optimized wine-making properties refer to improved strains with higher glycerol production and lower acetic acid production compared to the initial strain.

In another particular embodiment, optimized wine-making properties refer to improved strains with higher glycerol production, lower acetic acid production and lower ethanol production compared to the initial strain.

An additional object of the invention refers to a strain of *Saccharomyces cerevisiae* obtained by the previously described method deposited in the Spanish Type Culture Collection with number 13204 or mutants derived from the same where said mutants have the same or better optimized wine-making properties, than the CECT13204 strain.

The CECT 13204 strain has one or more mutations in the nucleotide sequence of the RTG2 gene and

where the glycerol production of said strain compared to the initial strain from which it comes is at least 60% (w/v) more, when a grape must vinification process is carried out and

where the acetic acid production of said strain compared to the initial strain from which it comes is at least 20% (w/v) less, when a grape must vinification process is carried out grape must vinification

where the ethanol production of said strain compared to the initial strain from which it comes is at least 5% (w/v) less, when a grape must vinification process is carried out.

A further object of the invention refers to a strain of *Saccharomyces cerevisiae* obtained by the previously described method deposited in the Spanish Type Culture Collection with number 13205 or mutants derived from the same where said mutants have the same or better optimized wine-making properties, than the CECT13205 strain.

The CECT 13205 strain has one or more mutations in the nucleotide sequence of the *RTG2* gene and where the glycerol production of said strain compared to the initial strain from which it comes is at least 60% (w/v) more, when a grape must vinification process is carried out and

where the acetic acid production of said strain compared to the initial strain from which it comes is at least 20% (w/v) less, when a grape must vinification process is carried out grape must vinification

where the ethanol production of said strain compared to the initial strain from which it comes is at least 5% (w/v) less, when a grape must vinification process is carried out.

An additional object of the invention refers to a strain of *Saccharomyces cerevisiae* obtained by the previously described method deposited in the Spanish Type Culture Collection with number 13206 or mutants derived from the same where said mutants have the same or better optimized wine-making properties, than the CECT13206 strain.

The CECT13206 strain has one or more mutations in the nucleotide sequence of the RTG2 gene and

where the glycerol production of said strain compared to the initial strain from which it comes is at least 60% (w/v) more, when a grape must vinification process is carried out and

where the acetic acid production of said strain compared to the initial strain from which it comes is at least 20% (w/v) less, when a grape must vinification process is carried out.

An additional object of the invention refers to a strain of *Saccharomyces cerevisiae* obtained by the previously described method deposited in the Spanish Type Culture Collection with number 13204, 13205, 13206 or mutants derived from the same where said mutants have optimized wine-making properties than strains 13204, 13205, 13206 respectively.

An additional object of the invention refers to the use of one or more strains defined above and/or obtained according to the method defined above for the production of alcoholic beverages through the alcoholic fermentation of one or more vegetable substrates.

In a particular embodiment, it is the use of one or more of the CECT13204, CECT13205 and CECT13206 strains for the production of alcoholic beverages through the alcoholic fermentation of one or more vegetable substrates.

In a particular embodiment it can be used for the vinification of grape must to obtain wine, champagne, cava, vermouth, fermentation of malt to obtain beer, fermentation of apple to obtain cider, mead, fermentation of one or more vegetable substrates to give rise to spirits, for example: whiskey, rum, patxaran, brandy (cognac, armagnac, sherry brandy, pisco), tequila, vodka, pomace or sake. Preferably for the vinification of grape must.

In another particular embodiment, the grape variety is selected between red grapes and/or white grapes.

In another particular embodiment the grape variety is selected from one or more of the group of red grapes consisting of: Abouriou, Acolon, Agiorgitiko, Aglianico, Alexandrouli, Alfrocheiro preto, Alicante bouschet, Alvarelhão, Ancellotta, Bábeascá neagrá, Barbera, Bastardo, Blauer portugieser, Blaufränkisch, Bobal, Bonarda, Bondola, Bovale, Brachetto, Bual, Cabernet franc, Cabernet sauvignon, Caíño tinto, Canaiolo, Cariñena, Carménére, Carnelian, Charbono, Cinsaut, Corvina, Croatina, Darkenusa, Dobricic, Dolcetto, Dornfelder, Durif, Feteascá neagrá, Freisa, Gamay, Garnacha, Graciano, Gropello, Grignolino, Hondarrabi beltza, Kadarka, Kratosija, Lagrein, Lambrusco, Listan negro, Malbec, Malvasia di Schierano, Malvasia negra, Mammolino, Mandilarla, Marattiko, Marzemino, Mavro, Mavrodafni, Mavrud, Mencia, Merlot, Millot, Misión, Molinara, Mónica, Montepulciano, Moscatel de Hamburgo, Monastrell, Mujuretuli, Nebbiolo, Négrette, Negroamaro, Ñero d'Avola, Okuzgozu, Ojaleshi, Oseleta, Petit verdot, Picapoll negro, Prieto picudo, Pinot meunier, Pinot noir, Piruleta, Pinotage, Plavac mali, Poulsard, Quebranta, Refosco, Rondinella, Rotberger, Ruby cabernet, Rubired, Ruché, Rufete, Sagrantino, Sangiovese, Saperavi, Schiava, Syrah, Sousón, St. Laurent, Tannat, Tamango, Tempranillo, Teroldego, Terret noir, Tinta baroque, Tinta cao, Touriga franca, Touriga nacional, Valdiguié, Vranac, Xinomavro, Zinfandel, Zweigelt, Valdepeñas,

In another particular embodiment the grape variety is selected from one or more of the group of white grapes consisting of: Alarije, Albalonga, Albana, Albariño, Albillo, Aledo, Aligoté, Altesse, Arinto, Arneis, Assyrtiko, Auxerrois blanc, Bogdanusa, Bacchus , Bical, Bouvier, Boal, Catarratto, Chardonnay, Centennia, Chasselas, Chenin blanc, Clairette, Colombard, Cortese, Courbu, Crouchen, Cserszegi füszeres, Doradillo, Erbaluce, Ehrenfelser, Ezerjó, Faber, Falanghina, Feteascá alba, Feteascá regala, Fiano , Flora, Folie blanche, Freisamer, Fromenteau, Furmint, Garganega, Garnacha blanca, Garnacha gris, Gewürztraminer, Glera, Gloria, Godello, Goldriesling, Grollam noir, Grüner veltliner, Hárslevelü, Hondarrabi zuri, Humagne blanche, Huxelrebe, Irsai olivér, Izsáki sarfehér, Jacquére, Pardina, Juhfark, Kerner, Krstac, Len de l'el, Loureira, Macabeo, Madeleine angevine, Malvar, Malvasia, Petit manseng, Gros manseng, Maria gomes, Marsanne, Mauzac, Melon de Burgundy, Merseguera, Moscatel de Alexandria, Muscat ottonel, Moscato giallo, Moschofilero, Mtsvane, Sauvignon vert, Müller-thurgau, Neuburger, Ondenc, Ortega, Pare, Palomino, Parellada, Pecorino, Petite arvine, Pedro Giménez, Pedro ximénez, Pinot blanc, Pinot grigio, Piquepoul blanc, Rabigato, Reichensteiner, Rhoditis, Riesling, Rieslaner, Rkatsiteli, Rotgipfler, Roussanne, Sauvignon blanc, Sauvignon gris, Savagnin blanc, Savagnin rosé, Savatiano, Scheurebe, Schónburger, Sémillon, Septiner, Cerceal, Siegerrebe, Sylvaner, Smederevka, Zierfandler, Sultanina, Symphony, Taminga, White Tempranillo, Torrontés, Trebbiano, Treixadura, Gray riesling, Verdejo, Verdia, Verdicchio, Verduzzo friulano, Verduzzo trevigiano, Vermentino, Vernaccia, Viognier, Macabeo, Welschriesling, Würzer, Xarello, Xynisteri, Zéta, Zeusz, Moscatel, Cardinal, Napoleon, Corinto, Noah, Isabella, Ivy, Concord, Niagara, Gewurstraminer, Zalema, Albarin and Viura

In a preferred embodiment, the grape is of the bobal variety,

Likewise, the invention comprises the following clauses

1. A method for obtaining an improved strain of *Saccharomyces cerevisiae* with optimized wine-making properties that comprises the following steps:

a) growing an initial strain of S. *cerevisiae* in a culture medium with a concentration of 2-aminoethyl-L-cysteine from 10 mg/l to 90 mg/l culture medium for at least 5 weeks and

b) isolate single mutants of the S. *cerevisiae* strain with optimized wine-making properties after the time of step a).

2. The method according to the preceding clause, wherein the required period of time is 6 weeks, more preferably at least 7 weeks, even more preferably at least 8 weeks, even more preferably at least 9 weeks, even more preferably at least 10 weeks.

3. The method according to clauses 1 or 2, wherein the amount of 2-aminoethyl-L-cysteine is between 13 mg/l and 85 mg/l, more preferably between 15 mg/l and 80 mg/l, even more preferably between 20 mg/l and 65 mg/l, even more preferably between 30 mg/l and 40 mg/l.

4. The method according to any one of the preceding clauses, where the required period of time is between 8 and 12 weeks and the amount of 2-aminoethyl-L-cysteine is between 30 mg/l and 40 mg/l.

5. The method according to any one of the preceding clauses, where the culture is continuous or discontinuous.

6. The method according to the previous clause, wherein when the culture is discontinuous, a fraction of said culture is inoculated into a fresh culture with fresh medium and 2-aminoethyl-L-cysteine when the OD(600nm) of the previous culture has reached an optical density OD(600nm) greater than 4.

7. The method according to any one of the preceding clauses, wherein the growth temperature of the culture is between 10°C and 60°C, preferably between 15°C and 50°C, more preferably between 20°C and 40°C and even more preferably between 25°C and 35°C.

8. The method according to any one of the preceding clauses, wherein the culture is at an agitation between 10 and 400 rpm, preferably between 50 and 300 rpm, more preferably between 100 and 250 rpm, more preferably between 150 and 220 rpm, and even more preferably between 160 and 200 rpm.

9. The method according to any one of the preceding clauses, wherein the culture medium is selected from SD, SC without lysine, synthetic must without lysine, or minimal medium without amino acids.

10. The method according to any one of the preceding clauses, where step b) is carried out by dripping serial dilutions on plates of selective medium with 2-AEC at a concentration of between 30 mg/l and 40 mg/l.

11. The method according to any one of the preceding clauses, wherein optimized wine-making properties refers to 2 or more of the following:

- higher glycerol production,
- lower acetic acid production, and
- lower ethanol production

compared to the initial strain from which it comes when a grape must vinification process is carried out.

12. The method according to the preceding clause, wherein optimized wine-making properties refers to higher glycerol production and lower acetic acid production compared to the initial strain from which it comes when a grape must vinification process is carried out.

13. The method according to clause 11, wherein optimized wine-making properties refers to higher glycerol production, lower acetic acid production and lower ethanol production compared to the initial strain from which it comes when a grape must vinification process is carried out.

14. The method according to any one of clauses 11 to 13, wherein the glycerol production of the strain with optimized wine-making properties compared to the initial strain from which it comes is at least 10% (w/v) more, preferably at least 20% (w/v) more when a grape must vinification process is carried out.

15. The method according to any one of clauses 11 to 13, wherein the ethanol production of the strain with optimized wine-making properties compared to the initial strain from which it comes is at least 0.5% (v/v) less, preferably 2% (v/v) less, more preferably at least 5% (v/v) less when a grape must vinification process is carried out.

16. The method according to any one of clauses 11 to 13, wherein the acetic acid production of the strain with optimized wine-making properties compared to the initial strain from which it comes is at least 0.5% (w/v) less, preferably 2% (w/v) less, more preferably at least 5% (w/v) less when a process is carried out of grape must vinification.

17. The method according to any one of the preceding clauses, wherein the *Saccharomyces cerevisiae* strain with optimized wine-making properties has one or more mutations in the nucleotide sequence of the RTG2 gene.

18. The method according to the preceding clause, wherein the mutation(s) in the nucleotide sequence of the RTG2 gene give rise to one or more of the following amino acid mutations of the resulting protein R30C; G248E and R560I.

19. The method according to any one of the preceding clauses, wherein the *Saccharomyces cerevisiae* strain with optimized wine-making properties has one or more mutations in the nucleotide sequence of the *LYS20* gene and/or the *LYS21* gene.

20. The method according to the preceding clause, wherein the mutation(s) in the nucleotide sequence of the *LYS20* gene and/or the *LYS21* gene give rise to one or more of the following amino acid mutations of the resulting protein N379D and R390G respectively.

21. The method according to any one of the preceding clauses 17 to 20, wherein the mutation is heterozygous or homozygous.

22. A strain of *Saccharomyces cerevisiae* with optimized wine-making properties obtained according to the method defined in one of clauses 1 to 21.

23. A strain of *Saccharomyces cerevisiae* with optimized wine-making properties, according to the preceding clause that has one or more mutations in the nucleotide sequence of the *RTG2* gene.

24. A strain of *Saccharomyces cerevisiae* with optimized wine-making properties, according to the preceding clause where the mutation(s) in the nucleotide sequence of the *RTG2* gene give rise to one or more of the following amino acid mutations of the resulting protein R30C; G248E and R560I.

25. A strain of *Saccharomyces cerevisiae* with optimized wine-making properties, according to any of clauses 21 to 23, having one or more mutations in the nucleotide sequence of the *LYS20* gene and/or the *LYS21* gene.

26. A strain of *Saccharomyces cerevisiae* with optimized wine-making properties, according to the previous clause where the mutation(s) in the nucleotide sequence of the *LYS20* gene and/or the *LYS21* gene give rise to one or more of the following amino acid mutations of the resulting protein N379D and R390G respectively.

27. A strain of *Saccharomyces cerevisiae* with optimized wine-making properties, according to any of clauses 21 to 26, where the mutation is heterozygous or homozygous.

28. A strain of *Saccharomyces cerevisiae* obtained according to the method defined in one of clauses 1 to 21, deposited in the Spanish Type Culture Collection with number 13204 or mutants derived from the same where said mutants have the same or better optimized wine-making properties as the CECT13204 strain.

29. A strain of *Saccharomyces cerevisiae* obtained according to the method defined in one of clauses 1 to 21, deposited in the Spanish Type Culture Collection with number 13205 or mutants derived from the same where said mutants have the same or better optimized wine-making properties as the CECT13205 strain.

30. A strain of *Saccharomyces cerevisiae* obtained according to the method defined in one of clauses 1 to 21, deposited in the Spanish Type Culture Collection with number 13206 or mutants derived from the same where said mutants have the same or better optimized wine-making properties as the CECT13206 strain.

31. A strain of *Saccharomyces cerevisiae* deposited in the Spanish Type Culture Collection with number 13204 or mutants derived from it where said mutants have the same or better optimized wine-making properties as the CECT13204 strain.

32. A strain of *Saccharomyces cerevisiae* deposited in the Spanish Type Culture Collection with number 13205 or mutants derived from it where said mutants have the same or better optimized wine-making properties as the CECT13205 strain.

33. A strain of *Saccharomyces cerevisiae* deposited in the Spanish Type Culture Collection with number 13206 or mutants derived from it where said mutants have the same or better optimized wine-making properties as the CECT13206 strain.

34. Use of the strain defined in one of clauses 22 to 33 and/or obtained according to the method defined in any one of clauses 1 to 21 for the production of alcoholic beverages through the alcoholic fermentation of one or more vegetable substrates.

35. Use according to the preceding clause where the alcoholic fermentation is selected between:

- vinification of grape must to obtain wine, champagne, cava, and/or vermouth

- fermentation of malt to obtain beer,

- fermentation of apple to obtain cider, and/or

- fermentation of one or more vegetable substrates to give rise to spirits, preferably for the vinification of grape must.

36. Use according to the previous clause, wherein the grape is selected between red grapes and/or white grapes

**Brief description of the figures**

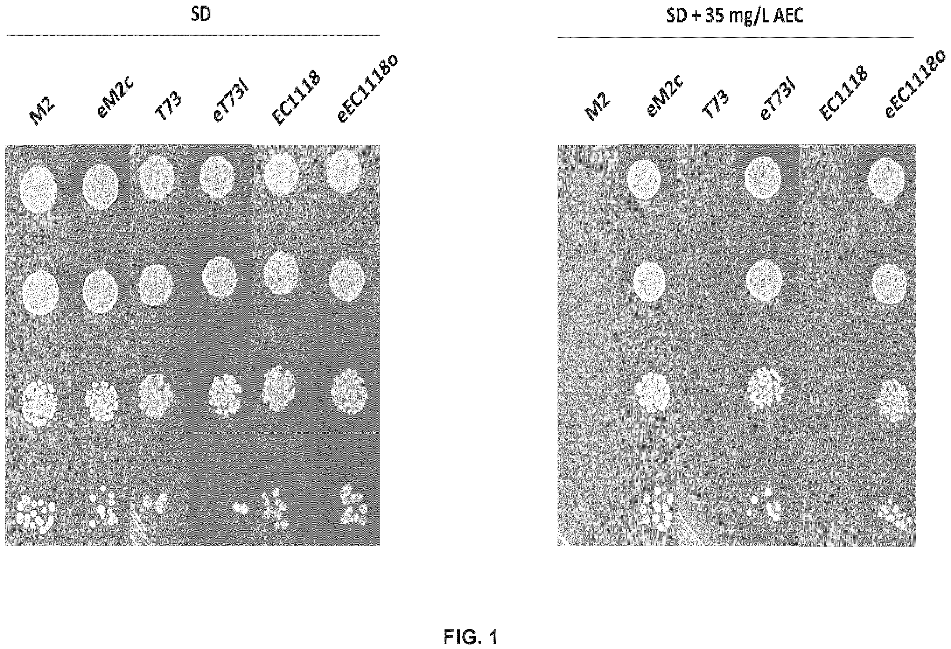

**Fig. 1. Tolerance of the evolved strains to AEC.** Drops of serial dilutions of a factor of magnitude at each step made in SD of the parental strains M2, T73 and EC1118 together with selected mutants obtained from adaptive evolution eM2c, eT73I and eEC1118o. 5 microlitre drops of each dilution are neatly deposited on a plate of minimal medium (SD, left) and SD with 35 mg/l 2-aminoethyl-L-cysteine (2-AEC), right.

**Fig, 2. Microvinifications of mutants isolated after adaptive evolution of the strain M2** A) Reducing sugars consumption curve of the strains evolved more than 150 generations from M2, during the fermentation process under vinification conditions in must of the bobal variety. Changes in the production of ethanol, glycerol and acetic acid in the must once fermented compared to the must fermented by the unevolved strain B)-D) respectively. Average values and standard deviations are shown. Experiments were performed in triplicate. * $p < 0.05$, Student's t-test, 2-tailed.

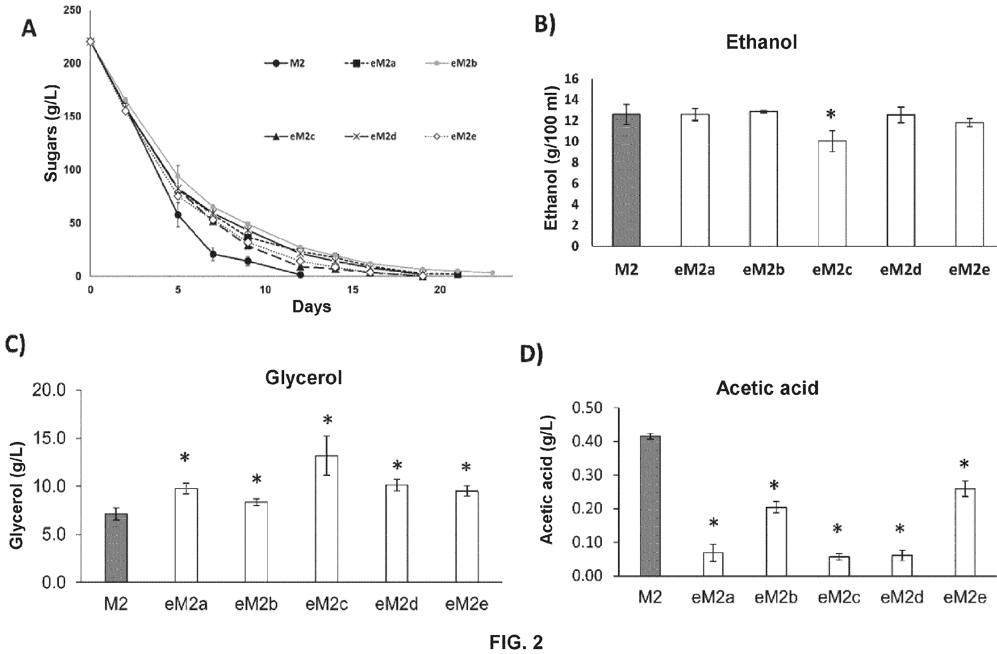

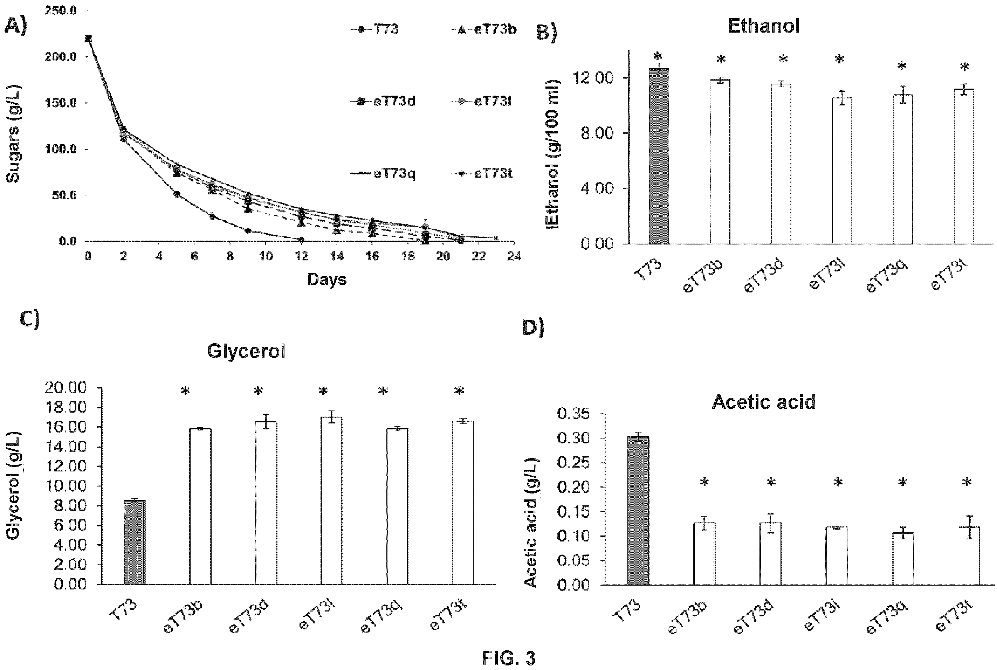

**Fig. 3. Microvinifications of mutants isolated after adaptive evolution of the strain T73.** Conditions as in Figure 2.

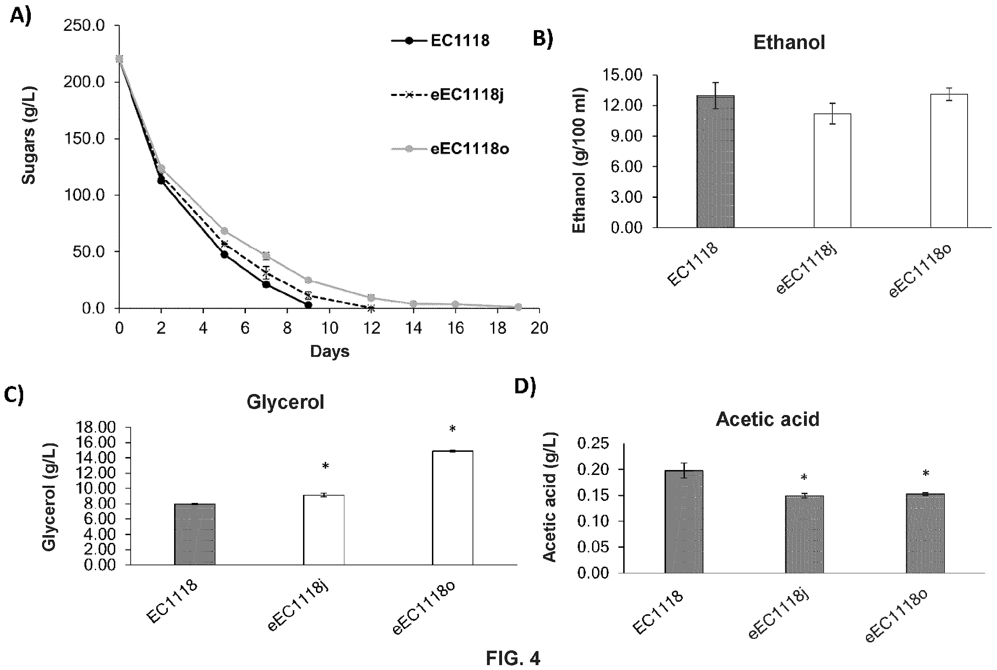

**Fig. 4. Microvinifications of mutants isolated after adaptive evolution of the strain EC1118.** Conditions as in Figure 2.

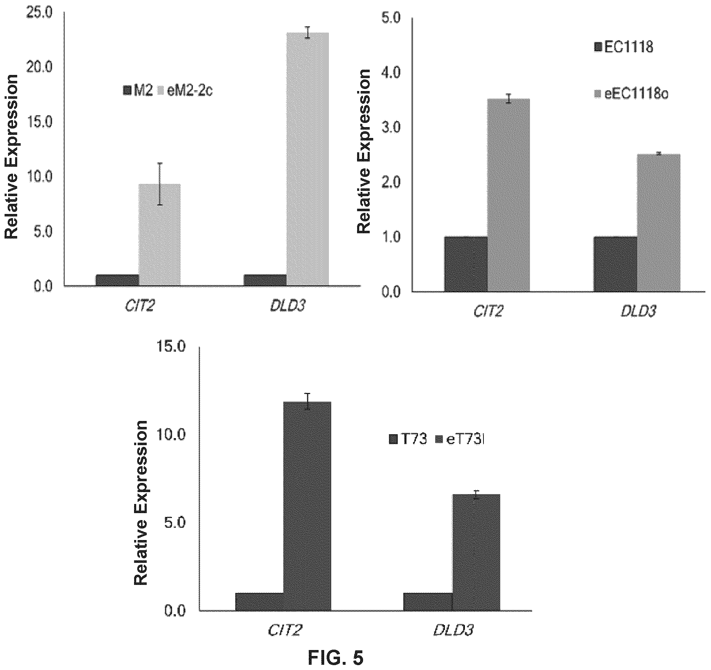

**Fig. 5. Analysis of the retrograde response.** Analysis of the expression of the retrograde response marker genes CIT2 and DLD3 in selected mutants and the initial strains. M2 left (initial strain, left vs improved strain, right), EC1118, center (initial strain, left vs improved strain, right) and T73 right (initial strain, left vs improved strain, right). Cells were grown in exponential phase in rich YPD medium. Normalization with respect to the *ACT1* actin gene.

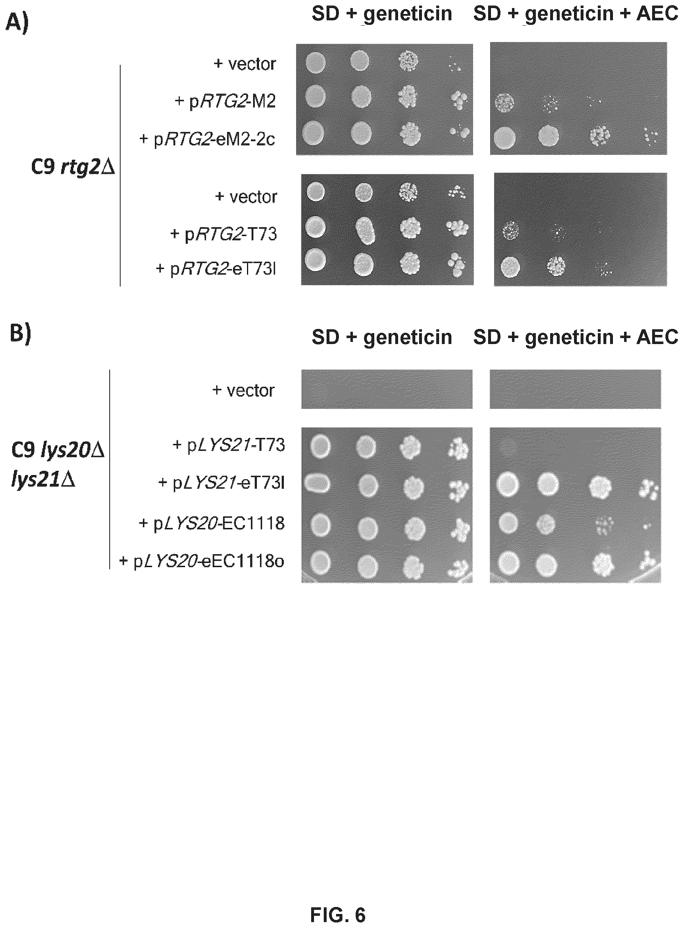

**Fig. 6. Subcloning of mutant alleles** A) Tolerance to 2-AEC of the haploid C9 *rtg2Δ* wine strain transformed with the centromeric plasmids containing the indicated wt and mutant alleles of *RTG2*. B) Tolerance to 2-AEC of the haploid C9 *lys20Δ lys21Δ* wine strain transformed with the centromeric plasmids containing the indicated wt and mutant alleles of *LYS20* and *LYS21*. Serial dilutions of a stationary culture were made in SD medium and 5 μl drops were deposited on SD and SD+AEC plates containing geneticin to select transformed strains.

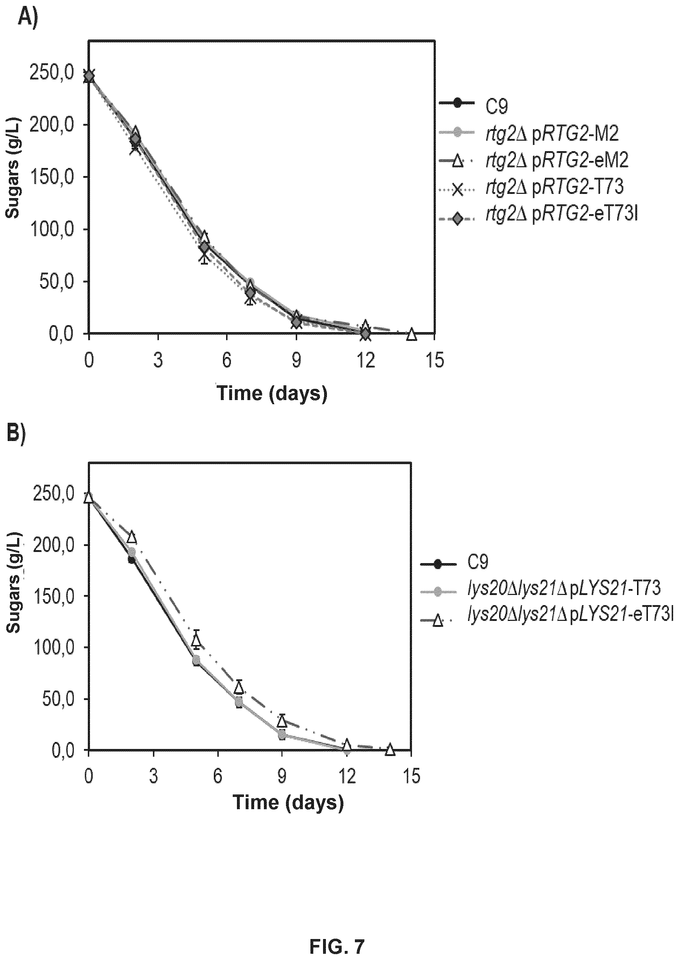

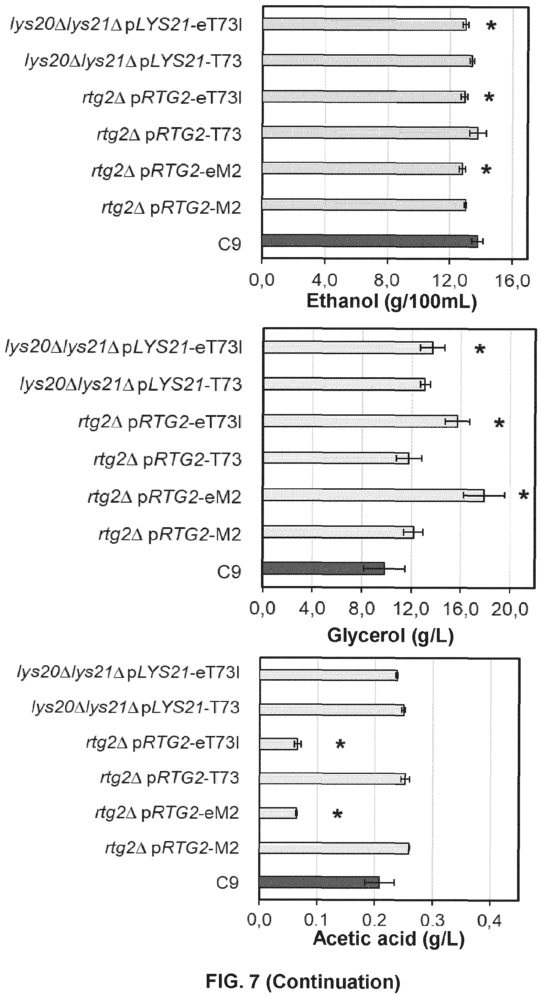

**Fig. 7. Microvinifications of the haploid C9 wine strain transformed with the mutant alleles of the RTG2 and LYS21 genes** A) Reducing sugars consumption curve of the C9 *rtg2Δ* strain, transformed with the *RTG2* alleles of the parental strains T73 and M2 (SEQ ID NO: 1) and after evolution, (SEQ ID NO: 2, eT73I and SEQ ID NO: 3, eM2c, respectively) during the fermentation process under vinification conditions in must of the bobal variety. B) Reducing sugars consumption curve of the C9 *lys20/lys21* strain, transformed with the *LYS21* alleles of the initial T73 strain (SEQ ID NO: 5) and evolved (SEQ ID NO: 6, eT73I), during the fermentation process under vinification conditions in must of the bobal variety. C) Changes in the production of ethanol, glycerol and acetic acid in the must once fermented compared to the must fermented by the haploid strain C9 with the alleles indicated above in panels A) and B), upper, middle and lower panels respectively. Average values and standard deviations are shown. The experiments were performed in triplicate. * $p < 0.05$, Student's t-test, 2-tailed.

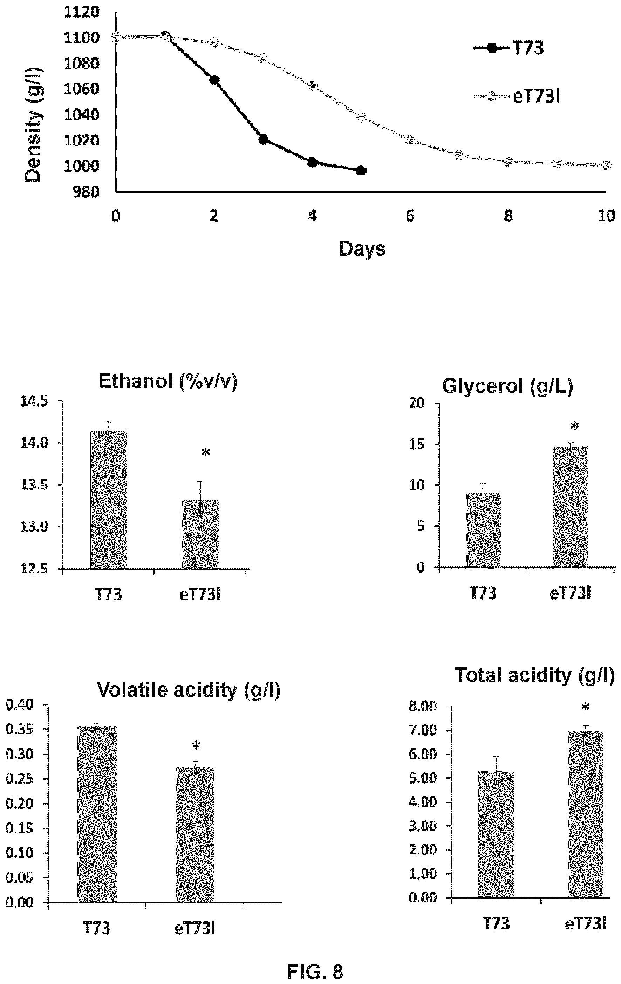

**Fig. 8. Vinifications in an experimental winery.** The evolution of the fermentation of the improved strain eT73I is shown in comparison to its initial strain T73 following the densitometric evolution of the vinification in 50 liters of Tempranillo must, as well as the final production of ethanol, glycerol, acetic acid (volatile acidity) and total acidity expressed as tartaric acid. Average values and standard deviations are shown. The experiments were performed in triplicate. * $p < 0.05$, Student's t-test, 2-tailed.

## Examples

Materials and methods

*Adaptive evolution in the presence of the toxic analogue of Lysine 2-AEC*

The different industrial strains of S. *cerevisiae* M2, T73 and EC1118 were used as initial strains for three independent directed evolution experiments. From an individual colony of each strain, a pre-culture of 5 mL of SD minimal medium (Yeast Nitrogen Base 0.17%, 0.5% ammonium sulfate, 2% glucose) was inoculated and grown for 24 hours at 30°C. From these pre-cultures, flasks containing 25 mL of SD supplemented with 35 mg/L or 174 μM 2-aminoethyl-L-cysteine (AEC) obtained from Sigma Aldrich (https://www.sigmaaldrich.com/ES/es/product/sigma/a2636) were inoculated at an initial OD(600nm) of 0.1. These cultures were grown in batch at 30°C with shaking (180 rpm), the final OD(600nm) of the culture was determined every 2-3 days and reinoculated in fresh medium at an initial OD(600nm) of 0.1. The adaptive evolution experiment was continued until 29 passages were completed (over a period of about 10 weeks), with more than 150 generations. Single mutants were isolated and tested for their tolerance to AEG by dropping serial dilutions onto selective medium plates.

*Determination of generations in the directed evolution method*

The determination of the number of generations was carried out from the data obtained from the optical density (600nm) of each of the passes performed.

$$\text{RATIO} = (\text{final OD600}) / (\text{initial OD600})$$

$$\text{Generations} = \log_2(\text{RATIO})$$

Accumulated generations = sum of generations

*AEC tolerance tests*

Individual colonies were grown to saturation on SD medium overnight. The optical density of the culture was determined at 600 nm to equalise the cultures and serial dilutions of one order of magnitude were made (100 $\mu$l of culture in 900 $\mu$l of sterile water) and 5 $\mu$l drops were deposited on SD and SD+ AEC 35 mg/l plates. The plates were incubated for two days at 30°C.

*Microvinifications in natural must*

In order to test the improvements in the vinification properties of the strains, microvinifications were carried out on must. The must used was red grape must from the Bobal variety from the 2015 harvest. In order to avoid contamination of the must once thawed, dimethyl dicarbonate (DMDC) 500 $\mu$g /L was added and left to act for 24 h in the cold. Experiments were carried out from liquid YPD pre-cultures from isolated mutant colonies grown for 48 h on a rotary shaker at 200 rpm. The musts were inoculated at a OD600 of 0.1. The vinifications were carried out in a volume of 30 ml at 24 °C with gentle agitation (50 rpm) in 30 ml disposable conical centrifuge tubes. Every 2-3 days, aliquots of the supernatant were taken and the fermentation was monitored following the consumption of reducing sugars, following the protocol of Robyt and Whelan, (1972) with certain modifications. 100 $\mu$l of a 1:100 dilution of the supernatant from vinification was mixed with one volume of the DNS reagent (dinitro-3,5-salicylic acid 0.01 g/ml, NaOH 16 mg/l, Na-K double tartrate 0.3 g/ml) and boiled for 5 min at 100 °C. Subsequently, 1 mL of distilled $H_2O$ was added to each reaction and the absorbance was measured at 540 nm. The sugar concentration was determined by interpolation with a standard line prepared from a 2 g/L glucose stock solution. At the end of the vinification the rest of the metabolites were measured. Ethanol was determined by enzymatic quantification using alcohol dehydrogenase. For this, 1 mL of Gly/Tris buffer containing the oxidised coenzyme of the reaction (0.2 M Glycine, Tris-HCl pH 9.7, 50 mM NAD+) is added to 200 $\mu$L of a 1:5000 dilution of the supernatant from the end of the vinification. The initial absorbance is measured at 340 nm. Next, 20 $\mu$L of alcohol dehydrogenase (20 U/mL, approx. 4 mg/L) is added and the absorbance is measured again after 15 min of incubation. The ethanol concentration is determined by interpolation of the standard line prepared from 1.2 mM ethanol. Acetic acid and glycerol were determined using Megazyme kits according to the manufacturer's instructions and were measured in a Variouskan multiwell plate reader.

**qPCR**

The amount of total yeast RNA was obtained by mechanical disruption with glass beads and selective precipitation with LiCl, using common protocols followed the field. The RNA was retrotranscribed according to the instructions of the *NZY First-Strand cDNA Synthesis Kit* and PCR was performed with *DLD3* and *CIT2* oligos, using the *ACT1* gene as a control in a Quantstudio 3 with the *NZYSpeedy qPCR Green Master Mix kit.* The oligonucleotides used in the PCR for the *ACT1, CIT2* and *DLD3* genes are described in the document Anna N. Starovoytova, Maxim I. Sorokin, Svyatoslav S. Sokolov, Fedor F. Severin, Dmitry A. Knorre. (2013) Mitochondrial signaling in Saccharomyces cerevisiae pseudohyphae formation induced by butanol, FEMS Yeast Research, 13, 367-374, https://doi.org/10.1111/ 1567-1364.12039, section Real-Time PCR

**Genome Sequencing**

The genome sequencing of the selected mutants was carried out by the Novogene company using common methods in the field.

The *Yea Star Genomic DNA kit (Zymo Research)* was used to extract genomic DNA from a 5 mL culture of yeast in YPD medium grown overnight following the manufacturer's recommended protocol. The quality and concentration of the extracted DNA were evaluated with the NanoDrop One spectrophotometer (Thermo Scientific). Sequencing, library preparation and subsequent bioinformatic analysis were performed by the company Novogene. Illumina-based pairwise sequencing with 150-bp reads was performed on libraries with 300-bp inserts with a resulting minimum coverage of 50X. The data were compared to the S288c genome (GenBank: GCF_000146045.2). Non-synonymous polymorphisms were identified in the CDS regions of selected genes *(RTG2, LYS20* and *LYS21).* Mutations existing in both the initial and evolved strains were excluded from further analyses.

**Cloning**

For cloning, the different alleles of the *RTG2* and *LYS21* genes were amplified by PCR from the genomic DNA of the initial strains and the selected evolved strains using Phusion DNA polymerase (Thermo Scientific). The PCR products and the pCUPI pNuiHA kanMX CEN plasmid were digested with the restriction enzymes Xhol and BamHI, gel-checked, and purified using the mi-PCR purification kit (Metabion). Ligation reactions were then carried out using T4 DNA ligase. Positive *E coli* transformants were selected, the plasmids were tested by PCR and transformed into yeast. C9 haploid wine strains with the respective chromosomal gene deleted were used in which RTG2 or *LYS20/LYS21* were used. Microvinifications in red grape juice were carried out as indicated above.

<u>Results</u>

*Obtaining by adaptive evolution in the presence* of *AEC of mutants with a phenotype compatible with the activation of the Retrograde Response*

Strains M2, EC1118 and T73 were grown in a continuous series of batch cultures in 25 ml of SD culture medium with 35 mg/l AEC. Reinoculations were carried out every 2/3 days from the previous culture in stationary phase to obtain a high number of generations, isolating individual colonies after 150 generations. Individual colonies were selected and tested for tolerance to AEC by drop plating (Figure 1, showing a representative mutant from each genetic background).
The most promising mutants were selected according to their tolerance to 2AEC to analyze their performance in natural must vinification. Figure 2 shows the mutants from strain M2. All mutants had a reduced rate of sugar consumption (glucose + fructose in the case of grape must) (Fig. 2A), but all finish vinification. This reduced speed can be associated with the alteration of carbon metabolism and the improvement of the rest of the qualities of the final product, an increase in the amount of glycerol and a decrease in ethanol and acetic acid, among others.
Regarding the production of metabolites (Figure 2B-D), there is a tendency towards lower ethanol production by the improved strains, although it is only significant in the case of the eM2c mutant where a reduction of up to 20% is observed in the production of ethanol. Regarding glycerol (Figure 2C), the mutants produce more glycerol during the grape must vinification process, particularly the eM2c strain. The effect on acetic acid is more pronounced (Figure 2D), with about half of the mutants showing a marked reduction in acetic acid to less than a quarter of that produced by the initial strain. The genotype of strain eM2c is *RTG2* R30C/ RTG2 R30C.

*Carrying out the method of the invention on the commercial strain* of *S. cerevisiae T73*

The method of the invention was carried out with the T73 strain in an identical manner as it was carried out with the M2 strain, (Figure 3). Very similar results were obtained: mutants that complete the fermentation or vinification of grape must with a slight delay compared to the initial strain (Figure 3A). All mutants obtained from T73 produce more glycerol and less acetic acid and reduce ethanol at the end of fermentation. For example, the mutant eT73I reduces it by 16%. The genotype of the improved strain eT73I is *RTG2* G248E/RTG2 G248E, *LYS21* R390G/LYS21 R390G. These results demonstrate that the method of the invention is reproducible

*Carrying out the method of the invention on the commercial strain* of S. *cerevisiae EC1118*

The method of the invention was carried out with the EC1118 strain in an identical manner as it was carried out with the M2 and T73 strains (Figure 4). From EC1118, the mutant eEC1118o was isolated with high glycerol production, lower acetic acid production, eEC1118j has a similar behavior, but to a lesser extent. Both mutants show a decreasing trend in ethanol production. Both mutants show a slowing down in the fermentation rate, but they complete it. The genotype of strain eEC1118o is *RTG2/ RTG2* R560I, *LYS20/LYS20* N379D, that is, heterozygous for both genes. These results confirm that the method of the invention is reproducible.
The following table (Table 1) shows the specific increase/decrease values of glycerol and acetic acid respectively obtained with the evolved strains of interest eM2c, eT73I and eEC1118o in comparison with their respective initial strains, the specific values have been obtained from panels C) and D) of Figures 2 -4

Table 1

| | Glycerol (g/L) | | | ratio | Acetic acid (g/L) | | | ratio | Ethanol (g/100mL) | | | ratio |
|---|---|---|---|---|---|---|---|---|---|---|---|---|
| | Average | ± | Std. Dev | | Average | ± | Std. Dev | | Average | ± | Std. Dev | |
| | | | | | | | | | | | | |
| M2 | 7,13 | ± | 0,64 | | 0,42 | ± | 0,01 | | 13,55 | ± | 0,41 | |
| eM2-2c | 13,18 | ± | 2,04 | 1,85 | 0,06 | ± | 0,01 | 0,14 | 12,3 | ± | 0,42 | 0,91 |

(continued)

| | Glycerol (g/L) | | | ratio | Acetic acid (g/L) | | | ratio | Ethanol (g/100mL) | | | ratio |
|---|---|---|---|---|---|---|---|---|---|---|---|---|
| | Average | ± | Std. Dev | | Average | ± | Std. Dev | | Average | ± | Std. Dev | |
| | | | | | | | | | | | | |
| T73 | 8,53 | ± | 0,15 | | 0,3 | ± | 0,01 | | 13,54 | ± | 0,06 | |
| eT73I | 16,92 | ± | 0,48 | 1,98 | 0,12 | ± | 0 | 0,4 | 11,66 | ± | 0,21 | 0,86 |
| | | | | | | | | | | | | |
| EC1118 | 7,97 | ± | 0,05 | | 0,2 | ± | 0,01 | | 13,82 | ± | 0,23 | |
| eEC1118 0 | 14,86 | ± | 0,1 | 1,86 | 0,15 | ± | 0 | 0,75 | 13,59 | ± | 0,09 | 0,98 |

In the case of the eEC1118o strain, it is observed that the increase in glycerol and the decrease in the levels of acetic acid and ethanol are lower than those obtained with the eM2c, eT73I strains. This may be because the mutation in RTG2 is heterozygous and therefore the observed phenotype is milder than with the other 2 strains that present homozygous mutations.

*Gene expression*

Expression by qPCR of the two typical retrograde response marker genes *CIT2* and *DLD3,* of the eM2c, eT73I and eEC1118o mutants was analyzed together with their initial strains (Figure 5). All showed an induction of both genes compared to the initial strain under exponential growth conditions in rich medium, where the expression of both genes is repressed, indicating an activation of the Retrograde Response attributable to their genotype.

In conclusion, the method of the invention provides a reproducible process for obtaining AEC-tolerant mutants with improved wine-making properties that behave with a phenotype compatible with the activation of the retrograde response and improve the organoleptic characteristics of the wine.

*Subcloning of genes of interest*

We proceeded to determine whether the mutations found in the improved strains are those that cause the AEC resistance phenotype, by subcloning the mutant alleles and analysing them phenotypically.

Regarding *RTG2*, the eM2c allele, *RTG2* R30C, and that of eT73I, *RTG2* G248E, were cloned in a single-copy centromeric plasmid pCUP1 pNuiHA kanMX CEN. These plasmids confer tolerance to AEC with respect to the plasmid containing the wild copy of the *RTG2* gene, by transforming the haploid wine strain without any chromosomal copy of the C9 *rtg2Δ* gene (Figure 6A).

The *LYS21* and *LYS20* genes are paralogous genes that encode homocitrate synthase, the first step of lysine biosynthesis. *LYS21* was amplified from the eT73I mutant and its initial strain T73 and introduced into the single-copy centromeric plasmid pCUP1 pNuiHA kanMX CEN and transformed into a haploid C9 *lys20Δ lys2lΔ* wine strain. Droplet assays on plates with AEC demonstrate that the *LYS21* R390G mutant allele increases tolerance to said compound, therefore demonstrating that said mutation is the cause of the phenotype (Figure 6B). The allele eEC1118o *LYS20* N379 D SEQ ID NO 8 and its control SEQ ID NO 8 were also cloned with the same result.

The effect of the mutations described in Figure 6 on the production of metabolites of oenological interest was then determined. The haploid strain *C9rtg2Δ* with the wild-type and mutant alleles of *RTG2* described in Figure 6A together with the wild-type strain C9 were grown in red grape must and vinification was followed by measuring sugar consumption (Figure 7A). The haploid strain C9*lys20Δlys21Δ* with the wild-type and mutant alleles of *LYS21* described in Figure 6B together with the wild-type C9 strain were grown in red grape must and vinification was followed by measuring sugar consumption (Figure 7B). All strains were able to finish the fermentation, so these mutations do not have a deleterious effect. Ethanol, acetic acid and glycerol were measured at the end of the fermentation (Figure 7C). The *RTG2* mutant alleles produced lower ethanol o and acetic acid production, and higher glycerol production by themselves, indicating that they are responsible for the phenotype of the eT73I and eM2c mutant strains. The effect of the *LYS21* allele of eT73I is smaller but goes in the same direction.

Winery experiments

Experimental fermentations were carried out with the mutant eT73I and its initial strain T73 in the experimental winery of Vitec, Wine Technology Center, Falset. The procedure is similar to microvinification, but adapted to a larger volume. Triplicate vinifications were used in 50 kg of Tempranillo grapes. The mutant strain has slower vinification kinetics, measured as density evolution (Figure 7), but completes fermentation. At the end of vinification the alcoholic strength has been reduced by 0.8% (v/v) (This is a decrease of 6% compared to the amount of ethanol of the initial strain 14.1% v/v vs 13.3% v/v), in addition to reducing volatile acidity (the measure of acetic acid). Glycerol has increased by 62% and total acidity measured as tartaric has also increased. In this way, the results observed in microvinification are confirmed on a large scale. The tasting of the wines by an expert panel indicated a slight improvement in the overall assessment of the wine produced by the eT73I strain compared to T73, so it does not represent a drop in the overall quality of the product.

The following table (Table 2) shows the specific values of increase/decrease of the values of glycerol and acetic acid respectively obtained with the evolved strains eT73I in comparison with the initial strain in a comparative experiment of fermentation in the winery, the specific values have been obtained from Figure 8.

Table 2

|  | Ethanol (g/100mL) | Gycerol (g/L) | Acetic acid (g/L) |
|---|---|---|---|
| T73 | 14,14 | 9,13 | 0,36 |
| eT73I | 13,33 | 14,77 | 0,27 |
| ratio | 0,9427157 | 1,6177437 | 0,75 |

**Sequence listing**

**SEQ_ID No 1:** RTG2 YGL252C SGDID:S000003221, Chromosome VII:25718..27484

**SEQ_ID No 2:** RTG2 eT73I

**SEQ_ID No 3:** RTG2 eM2c

**SEQ_ID No 4:** RTG2 eEC1118o

**SEQ_ID No 5:** LYS21 YDL131W SGDID:S000002289, Chromosome IV:227393..228715

**SEQ_ID No 6:** LYS21 YDL131 W eT73I

**SEQ_ID No 7:** LYS20 YDL182W SGDID:S000002341, Chromosome IV:133437..134723

**SEQ_ID No 8**: LYS20 eEC1118o

**Claims**

**1.** A method for obtaining a mutant strain of *Saccharomyces cerevisiae* with optimized wine-making properties that comprises the following steps:

a) growing an initial strain of *S. cerevisiae* in a culture medium with a concentration of 2-aminoethyl-L-cysteine from 10 mg /l to 90 mg/l of culture medium for at least 5 weeks and
b) isolate single mutants of the *S. cerevisiae* strain with optimized wine-making properties after the time of step a),

where optimized wine-making properties refers to greater glycerol production and lower acetic acid production compared to the initial strain from which it comes when a grape must vinification process is carried out.

**2.** The method according to the preceding claim, wherein the required period of time is 6 weeks, more preferably at least 7 weeks, even more preferably at least 8 weeks, even more preferably at least 9 weeks, even more preferably at least 10 weeks.

3. The method according to claims 1 or 2, wherein the amount of 2-aminoethyl-L-cysteine is between 13 mg/l and 85 mg/l, more preferably between 15 mg/l and 80 mg/l, even more preferably between 20 mg/l and 65 mg/l, even more preferably between 30 mg/l and 40 mg/l.

4. The method according to any one of the preceding claims, where the required period of time is between 8 and 12 weeks and the amount of 2-aminoethyl-L-cysteine is between 30 mg/l and 40 mg/l.

5. The method according to any one of the preceding claims, where the culture is continuous or discontinuous.

6. The method according to the previous claim, wherein when the culture is discontinuous, a fraction of said culture is inoculated into a fresh culture with fresh medium and 2-aminoethyl-L-cysteine when the OD(600nm) of the previous culture has reached an optical density OD(600nm) greater than 4.

7. The method according to any one of the preceding claims, wherein the growth temperature of the culture is between 10°C and 60°C, preferably between 15°C and 50°C, more preferably between 20°C and 40°C and even more preferably between 25°C and 35°C.

8. The method according to any one of the preceding claims, wherein the culture is at an agitation between 10 and 400 rpm, preferably between 50 and 300 rpm, more preferably between 100 and 250 rpm, more preferably between 150 and 220 rpm, and even more preferably between 160 and 200 rpm.

9. The method according to any one of the preceding claims, wherein the culture medium is selected from SD, SC without lysine, synthetic must without lysine, or minimal medium without amino acids.

10. The method according to any one of the preceding claims, where step b) is carried out by dripping serial dilutions on plates of selective medium with 2-AEC at a concentration of between 30 mg/l and 40 mg/l.

11. The method according to claim 1, wherein optimized wine-making properties refers to higher glycerol production, lower acetic acid production and lower ethanol production compared to the initial strain from which it comes when a grape must vinification process is carried out.

12. The method according to any of the preceding claims, wherein the glycerol production of the strain with optimized wine-making properties compared to the initial strain from which it comes is at least 10% (w/v) more, preferably at least 20% (w/v) more when a grape must vinification process is carried out.

13. The method according to claim 11, wherein the ethanol production of the strain with optimized wine-making properties compared to the initial strain from which it comes is at least 0.5% (v/v) less, preferably 2% (v/v) less, more preferably at least 5% (v/v) less when a grape must vinification process is carried out.

14. The method according to any of the preceding claims, wherein the acetic acid production of the strain with optimized wine-making properties compared to the initial strain from which it comes is at least 0.5% (w/v) less, preferably 2% (w/v) less, more preferably at least 5% (w/v) less when a process is carried out of grape must vinification.

15. A strain of *Saccharomyces cerevisiae* with optimized wine-making properties obtained according to the method defined in any of claims 1 to 14 that has one or more mutations in the nucleotide sequence of the *RTG2* gene and

     where the glycerol production of said strain compared to the initial strain from which it comes is at least 60% (w/v) more, when a grape must vinification process is carried out,
     where the acetic acid production of said strain compared to the initial strain from which it comes It is at least 20% (w/v) less, when a grape must vinification process is carried out.

16. A strain of *Saccharomyces cerevisiae* with optimized wine-making properties obtained according to the method defined in one of claims 1 to 14 or according to the previous claim that has one or more mutations in the nucleotide sequence of the *RTG2* gene and

where the glycerol production of said strain compared to the initial strain from which it comes is at least 60% (w/v) more, when a grape must vinification process is carried out,

where the acetic acid production of said strain compared to the initial strain from which it comes is at least 20% (w/v) less, when a grape must vinification process is carried out, and

where the ethanol production of said strain compared to the initial strain from which it comes It is at least 5% (w/v) less, when a grape must vinification process is carried out.

17. A strain of *Saccharomyces cerevisiae* with optimized wine-making properties according to the preceding claim, wherein the mutation(s) in the nucleotide sequence of the *RTG2* gene give rise to one or more of the following amino acid mutations of the resulting protein R30C; G248E and R560I.

18. A strain of *Saccharomyces cerevisiae* with optimized wine-making properties according to any of the claims 15 to 17, having one or more mutations in the nucleotide sequence of the *LYS20* gene and/or the *LYS21* gene.

19. A strain of *Saccharomyces cerevisiae* with optimized wine-making properties according to the preceding claim, wherein the mutation(s) in the nucleotide sequence of the *LYS20* gene and/or the *LYS21* gene give rise to one or more of the following amino acid mutations of the resulting protein N379D and R390G respectively.

20. A strain of *Saccharomyces cerevisiae* with optimized wine-making properties according to any of the claims 15 to 19, wherein the mutation is heterozygous or homozygous.

21. A strain of *Saccharomyces cerevisiae* obtained according to the method defined in any of claims 1 to 14, deposited in the Spanish Type Culture Collection with number 13204 or mutants derived from the same where said mutants have the same or better optimized wine-making properties as the CECT13204 strain.

22. A strain of *Saccharomyces cerevisiae* obtained according to the method defined in any of claims 1 to 14, deposited in the Spanish Type Culture Collection with number 13205 or mutants derived from the same where said mutants have the same or better optimized wine-making properties as the CECT13205 strain.

23. A strain of *Saccharomyces cerevisiae* obtained according to the method defined in any of claims 1 to 14, deposited in the Spanish Type Culture Collection with number 13206 or mutants derived from the same where said mutants have the same or better optimized wine-making properties as the CECT13206 strain.

24. A strain of *Saccharomyces cerevisiae* deposited in the Spanish Type Culture Collection with number 13204 or mutants derived from it where said mutants have the same or better optimized wine-making properties as the CECT13204 strain.

25. A strain of *Saccharomyces cerevisiae* deposited in the Spanish Type Culture Collection with number 13205 or mutants derived from it where said mutants have the same or better optimized wine-making properties as the CECT13205 strain.

26. A strain of *Saccharomyces cerevisiae* deposited in the Spanish Type Culture Collection with number 13206 or mutants derived from it where said mutants have the same or better optimized wine-making properties as the CECT13206 strain.

27. Use of the strain defined in any of claims 15 to 26 and/or obtained according to the method defined in any one of clauses 1 to 14 for the production of alcoholic beverages by alcoholic fermentation of one or more vegetable substrates.

28. Use according to the preceding claim wherein the alcoholic fermentation is selected between:

   - vinification of grape must to obtain wine, champagne, cava, and/or vermouth
   - fermentation of malt to obtain beer,
   - fermentation of apple to obtain cider, and/or
   - fermentation of one or more vegetable substrates to produce spirits, preferably for the vinification of grape must.

29. Use according to the previous claim, wherein the grape is selected between red grapes and/or white grapes

**FIG. 1**

FIG. 2

EP 4 534 646 A1

FIG. 3

FIG. 4

FIG. 5

A)

SD + geneticin    SD + geneticin + AEC

C9 *rtg2Δ*

+ vector
+ p*RTG2*-M2
+ p*RTG2*-eM2-2c

+ vector
+ p*RTG2*-T73
+ p*RTG2*-eT73I

B)

SD + geneticin    SD + geneticin + AEC

C9 *lys20Δ*
*lys21Δ*

+ vector
+ p*LYS21*-T73
+ p*LYS21*-eT73I
+ p*LYS20*-EC1118
+ p*LYS20*-eEC1118o

FIG. 6

**FIG. 7**

FIG. 7 (Continuation)

FIG. 8

# INTERNATIONAL SEARCH REPORT

| International application No. |
| --- |
| PCT/ES2023/070344 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
C12N, C12G, C12R

Documentation searched other than minimum documentation to the extent that such documents are included in the fields searched

Electronic data base consulted during the international search (name of data base and, where practicable, search terms used)

EPODOC, INVENES, WPI, INTERNET, BIOSIS, MEDLINE, CAPLUS, BD-TXTE

**C. DOCUMENTS CONSIDERED TO BE RELEVANT**

| Category* | Citation of document, with indication, where appropriate, of the relevant passages | Relevant to claim No. |
| --- | --- | --- |
| A | EP 2902481 A1 (DANSTAR FERMENT AG et al.) 05/08/2015, claims; tables. | 1-29 |
| A | EP 2634247 A1 (GUSERBIOT S L U) 04/09/2013, tables | 15-29 |
| A | VALLEJO, B. Análisis de las rutas de señalización de nutrientes en cepas vínicas de *Saccharomyces cerevisiae* en condiciones de vinificación. Tesis doctoral. Departament of Bioquímica i Biologia Molecular. Universidad of Valencia, 2019, paragraph 3.7, pages 227-237 Retrieved from Internet URL: https://roderic.uv.es/handle/10550/72839 | 15-29 |

☒ Further documents are listed in the continuation of Box C.   ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 10/08/2023 | **(14/08/2023)** |
| Name and mailing address of the ISA/ | Authorized officer |
| | A. Polo Diez |
| OFICINA ESPAÑOLA DE PATENTES Y MARCAS Paseo de la Castellana, 75 - 28071 Madrid (España) Facsimile No.: 91 349 53 04 | Telephone No. 913495524 |

Form PCT/ISA/210 (second sheet) (July 2022)

**INTERNATIONAL SEARCH REPORT**

| International application No. |
| --- |
| PCT/ES2023/070344 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| A | RIOS-ANJOS R. M.et al. Structural and functional mapping of Rtg2p determinants involved in retrograde signaling and aging of *Saccharomyces cerevisiae*. Plos One United States, 2017, vol. 12 (5) pages e0177090, ISSN 1932-6203 (Electronic), DOI:10.1371/journal.pone.0177090 pubmed:28472157 | 15-26 |
| A | GASENT-RAMIREZ J. M et al. Lysine-overproducing mutants of *Saccharomyces cerevisiae* Baker´s yeast isolated in continuous culture. Applied And Environmental Microbiology, 1997, vol. 63 (12), pages 4800-4806, ISSN 0099-2240, material and methods | 1-14 |

Form PCT/ISA/210 (continuation of second sheet) (July 2022)

| | | International application No. |
|---|---|---|
| | | PCT/ES2023/070344 |

| Patent document cited in the search report | Publication date | Patent family member(s) | Publication date |
|---|---|---|---|
| EP2902481 A1 | 05.08.2015 | NZ753744 A | 29.07.2022 |
| | | ZA201601951 B | 25.05.2022 |
| | | US2021017613 A1 | 21.01.2021 |
| | | CL2016001883 A1 | 06.01.2017 |
| | | US2016348192 A1 | 01.12.2016 |
| | | US10829827 B2 | 10.11.2020 |
| | | AU2015212752 A1 | 19.05.2016 |
| | | AU2015212752B B2 | 01.04.2021 |
| | | EP3099780 A1 | 07.12.2016 |
| | | WO2015114115 A1 | 06.08.2015 |
| EP2634247 A1 | 04.09.2013 | ES2525343T T3 | 22.12.2014 |

## INTERNATIONAL SEARCH REPORT

International application No.

PCT/ES2023/070344

CLASSIFICATION OF SUBJECT MATTER

*C12N1/18* (2006.01)
*C12N1/38* (2006.01)
*C12N15/01* (2006.01)
*C12G1/02* (2006.01)
*C12G3/02* (2019.01)
*C12R1/865* (2006.01)

Form PCT/ISA/210 (extra sheet) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10829827 B2

**Non-patent literature cited in the description**

- **MATALLANA E** ; **ARANDA A**. Biotechnological impact of stress response on wine yeast. *Lett Appl Microbiol.*, 2017, vol. 64 (2), 103-110
- Stress Response in Yeasts Used for Food Production. **OROZCO** ; **MATALLANA** ; **ARANDA**. Food Molecular Microbiology. Taylor & Francis, 2019
- Saccharomamyces cerevisiae.. *Molec Gen. Genet.*, 1985, vol. 200, 291-294, https://doi.org/10.0007/BF00425438
- **BEATRIZ VALLEJO ESTARÁ**. *Analysis of nutrient signaling pathways in wine strains of Saccharomyces cerevisiae under winemaking conditions*, 2019
- **GASENT-RAMÍREZ JM** ; **BENITEZ T**. Lysine-over-producing mutants of Saccharomyces cerevisiae baker's yeast isolated in continuous culture.. *Appl Environ Microbiol.*, 1997, vol. 63 (12), 4800-4806
- **XU, X.** ; **WILLIAMS, T. C.** ; **DIVNE, C.** ; **PRETORIUS, I. S.** ; **PAULSEN, I. T.** Evolutionary engineering in Saccharomyces cerevisiae reveals a TRK1-dependent potassium influx mechanism for propionic acid tolerance.. *Biotechnol Biofuels*, 2019, vol. 12, 97, https://doi.org/10.1186/s13068-019-1427-6
- **DYMOND JS**. Saccharomyces cerevisiae growth media.. *Methods Enzymol.*, 2013, vol. 533, 191-204
- **ANNA N. STAROVOYTOVA** ; **MAXIM I. SOROKIN** ; **SVYATOSLAV S. SOKOLOV** ; **FEDOR F. SEVERIN** ; **DMITRY A. KNORRE**. Mitochondrial signaling in Saccharomyces cerevisiae pseudohyphae formation induced by butanol. *FEMS Yeast Research*, 2013, vol. 13, 367-374, https://doi.org/10.1111/1567-1364.12039